# EUROPÄISCHE PATENTSCHRIFT

(11) **EP 1 789 504 B1**
(45) Veröffentlichungstag und Bekanntmachung des Hinweises auf die Patenterteilung: **22.04.2009**
(21) Anmeldenummer: 05777183.4
(22) Anmeldetag: 06.09.2005
(51) Int. Cl.: C09D 11/00, C03C 4/00

(54) **TÄTOWIERFARBE**
TATTOOING INK
ENCRE DE TATOUAGE

(30) Priorität: 07.09.2004 DE 202004014053 U
(43) Veröffentlichungstag der Anmeldung: 30.05.2007
(73) Patentinhaber: Deep Colours! GmbH, 76776 Neuburg am Rhein (DE)
(72) Erfinder: MICHEL, Ralf, 76829 Landau (DE)
(74) Vertreter: Petirsch, Markus
(86) Internationale Anmeldenummer: PCT/EP2005/009537
(87) Internationale Veröffentlichungsnummer: WO 2006/027200

(56) Entgegenhaltungen:
- WO-A-99/07777
- US-A- 6 013 122
- ROETHER J A ET AL: "Development and in vitro characterisation of novel bioresorbable and bioactive composite materials based on polylactide foams and Bioglass<(>R) for tissue engineering applications" BIOMATERIALS, ELSEVIER SCIENCE PUBLISHERS BV., BARKING, GB, Bd. 23, Nr. 18, September 2002 (2002-09), Seiten 3871-3878, XP004372499 ISSN: 0142-9612

## Beschreibung

Die Erfindung betrifft eine Tätowierfarbe umfassend Wasser, Farbpigmente und ein Bindemittel zur Verbindung der Farbpigmente mit dem Wasser.

Ursprünglich wurde zum Zwecke des Tätowierens auf archaische Weise die Haut eingeschnitten und anschließend die Wunde mit Tinte, Asche oder anderen Farbstoffen gefärbt. Heute wird die Farbe durch eine Nadel in die Haut eingebracht, um ein Bild oder einen Text auf der Haut dauerhaft sichtbar zu machen. Gleichzeitig mit dem Durchstechen der Haut wird der Farbstoff in die Haut eingebracht. Der Stich darf weder zu oberflächlich sein, noch darf er zu tief eindringen. Wird der Farbstoff lediglich in die äußerste Hautschicht, also in die Zelllagen der Epidermis, eingebracht, werden die Farbteilchen bei der fortwährenden Erneuerung der Hautschicht ausgewaschen und abgestoßen. Wird der Farbstoff zu tief in die Haut gebracht, kommt es zu Blutungen, die ein Auswaschen der Farbe aus der Haut bewirken.

Bei allen Arten des Tätowierens wird jedoch die Haut verletzt, wenn eine Farbe in bzw. unter die Haut eingespritzt wird. Dabei kann es zu Entzündungen kommen. Es ist deshalb wichtig, nicht nur auf eine besondere Sauberkeit zu achten und sterilisierte Geräte zu benutzen, sondern auch Farben mit bestimmten Eigenschaften zu verwenden, die das Risiko einer Entzündung verringern.

Bis gegen Ende des 20. Jahrhunderts wurden vorwiegend Farben verwendet, die aus Wasser, Alkohol, Glycerin, teilweise Rosenwasser und aus Farbpigmenten bestanden. Moderne Farben sind in der Regel frei von Alkoholen. Sie basieren auf einer wässrigen Lösung, die Pigmente als Farbteile enthält.

Neben besonderen Tätowierfarben wird heute auch noch Tinte zur Tätowierung verwendet, wie es schon seit Jahrzehnten praktiziert wird. Die Tinte besteht ebenfalls aus Farbpigmenten und Wasser. Um die hydrophoben Eigenschaften der Pigmente zu überwinden, werden bei allen modernen Farben zusätzlich Bindemittel eingesetzt. Damit wird die stoffliche Trennung von Wasser und Pigment aufgehoben, so dass das Pigment im Wasser dispergiert wird.

Als Bindemittel wird häufig Schellack, teilweise in Kombination mit weiteren Substanzen, eingesetzt. Auch Tinte enthält typischerweise Schellack als Bindemittel. Bei Tätowierfarben wird Schellack stets mit weiteren Substanzen kombiniert. Bei schwarzer Tätowierfarbe wird meist eine Mischung aus Schellack und Borax zugegeben. Dies ist eine preiswerte Mischung, die für einfache Farben ausreicht. Hochwertige Tätowierfarben und bunte Farben basieren dagegen häufig auf einer Kombination aus Schellack und Ammoniak. Darüber hinaus sind als Bindemittel auch so genannte Povidone bekannt, die zur Viskositätsregulierung als Verbundmittel dienen.

Den modernen Tätowierfarben qualifizierter Hersteller ist gemeinsam, dass sie eine gute Farb- bzw. Lichtechtheit aufweisen. Sie sind auch insoweit gesundheitsverträglich, als sie auf Schwermetallbelastungen geprüft sind und keine krebserzeugenden aromatischen Amine aufweisen.

Aufgabe ist es, eine hochwertige, leicht zu verarbeitende Tätowierfarbe mit guter Lichtechtheit bereitzustellen, die antimikrobiell, entzündungshemmend und verträglichkeitsoptimiert ist.

Gelöst wird die Aufgabe durch eine Tätowierfarbe mit den Merkmalen gemäß des Schutzanspruchs 1.

Die erfindungsgemäße Tätowierfarbe ist zusammengesetzt aus Wasser, Farbpigmenten, einem Bindemittel und als weiterem Bestandteil bioaktives Glas. Das in der Tätowierfarbe enthaltene Wasser ist in der Regel demineralisiertes oder destilliertes Wasser. Die Pigmente weisen unterschiedliche Farben auf, so dass sich fast alle Farbtöne von weiß bis schwarz herstellen lassen.

Das bioaktive Glas liegt in relativ geringen Anteilen in der Tätowierfarbe vor. Vorwiegend basiert das Glas auf einem Gemisch, das überwiegend Siliziumdioxid enthält. Als weitere Bestandteile können Oxide der Alkali-Metalle und der Erdalkali-Metalle vorliegen. Darüber hinaus können zusätzliche Bestandteile in dem Glasgemisch vorhanden sein. Das bioaktive Glas hat die Wirkung, entzündungshemmend zu sein. Das Auftreten und eine mögliche Ausbreitung von Entzündungen, die beim Eindringen der Tätowiernadel in die Haut entstehen können, werden dadurch verhindert. Darüber hinaus wird die Wundheilung gefördert. Der Heilungsprozess der durch die Tätowiernadel verletzten Haut wird durch das in der Farbe enthaltene Glas beschleunigt. Auch werden Hautreizungen durch die beigemengten Glaspartikel verringert und klingen sehr schnell ab.

Neben diesen positiven Einflüssen der Glaspartikel als Bestandteil der erfindungsgemäßen Tätowierfarbe werden im übrigen die Farbeigenschaften wie Lichtechtheit und Farbechtheit durch das Glas nicht beeinträchtigt. Das Glas selbst ist sehr gut verträglich, da es ein Stoff ist, auf den selbst sensible Haut nicht mit Reizungen reagiert. Das Glas ruft auch sonst keine Reaktionen der Haut oder gar Entzündungen hervor. Insgesamt liegt also eine Tätowierfarbe vor, die, bis auf die Pigmente, frei von sensibilisierenden Inhaltsstoffen ist.

Die Glaspartikel in der Tätowierfarbe haben ferner eine antimikrobielle Wirkung. Eine Keimbildung in der Tätowierfarbe wird somit zuverlässig verhindert. Dies ist besonders wichtig, wenn aus einer Farbflasche mehr als einmal Tätowierfarbe ertnommen wird. Darüber hinaus ist die Wirksamkeit gegen einige Schimmelpilze und Hefen wissenschaftlich erwiesen. Nach dem Öffnen der Flasche und nach der ersten Entnahme von Tätowierfarbe können nämlich Bakterien in die Farbe gelangen. Aufgrund der antimikrobiellen Wirkung des Glases werden die Bakterien jedoch abgetötet. Auch wenn aus einer Farbflasche mehrfach Farbe entnommen wird, entstehen keine gesundheitliche Risiken. Die Lagerung von angebrochenen Farbflaschen stellt ebenfalls kein Problem mehr dar.

In einer bevorzugten Ausführung der Tätowierfarbe ist das Bindemittel zur Verbindung der Farbpigmente mit dem Wasser ein Povidon. Dabei wird insbesondere Polyvinyl-Pyrrolidon verwendet. Dieses Bindemittel sorgt für eine sehr gute Verknüpfung der Farbpigmente mit dem Wasser. Durch das Povidon werden im Wasser die Strukturen geschaffen, die die Farbpigmente quasi in Schwebe halten.

Besonders vorteilhaft ist eine Tätowierfarbe, deren Bestandteile in der folgenden Zusammensetzung vorliegen: Die Farbe weist einen Anteil von 35 Gew% bis 65 Gew% Wasser auf. Die Pigmente liegen in einem Anteil von 1 Gew% bis 40 Gew% vor. Ein Povidon ist mit 5 Gew% bis 20 Gew% Anteil vertreten; Crospovidone liegen mit einem Anteil von 0,1 Gew% bis 2,5 Gew% vor. Die Glaspartikel haben einen Anteil an der Tätowierfarbe von 0,1 Gew% bis 10 Gew%.

Die Zusammensetzung der Tätowierfarbe variiert in Abhängigkeit von der farblichen Zusammenstellung. So enthält schwarze Tätowierfarbe einen Farbpigmentanteil von 8 Gew% bis 16 Gew%. Eine weiße Tätowierfarbe weist einen Pigmentanteil zwischen 35 Gew% bis 40 Gew% auf. Der Anteil der verwendeten Povidone liegt meistens im Bereich von 7,5 Gew% bis 17,5 Gew%, wobei ein Povidon-Anteil von 17,5 Gew% bevorzugt ist. Ebenso hat sich eine Tätowierfarbe mit einem Anteil von 2,5 Gew% Crospovidonen als besonders vorteilhaft erwiesen. Crospovidone sind Substanzen, die eine Verklumpung und Ablagerung der Pigmente verhindern. Sowohl der Anteil an Povidonen als auch an Crospovidonen hängt von dem Anteil der verwendeten Farbpigmente und auch von dem Farbton der Tätowierfarbe ab. Eine schwarze Farbe weist deshalb geringere Anteile an Povidon und Crospovidon auf als eine weiße Farbe, die einen höheren Pigmentanteil hat.

In einer vorteilhaften Ausführungsform enthält die Tätowierfarbe ein Glas, das Bestandteile an Siliziumdioxid im Bereich 10 Gew% bis 90 Gew% hat. Als weitere Bestandteile liegen als Oxid der Erdalkali-Metalle Kaliumoxid CaO im Bereich von 0,1 Gew% bis 50 Gew% und als Oxid der Alkali-Metalle Natriumoxid Na₂O im Bereich von 0,1 Gew% bis 60 Gew% vor. Weiter ist dem Gemisch eine Form vom Phosphoroxid, insbesondere P₂O₅, mit einem Anteil von 0,1 Gew% bis 40 Gew% beigemengt.

Besonders vorteilhaft ist eine Tätowierfarbe, bei der das Glas eine Zusammensetzung von 45 Gew% Siliciumdioxid, 24,5 Gew% Calciumoxid, 24,5 Gew% Natriumoxid und 6 Gew% Phosphoroxid enthält. Ein solches bioaktives Glas wird von der Firma SCHOTT GLAS hergestellt und unter dem Handelsnamen "Vitryxx" geführt. Es handelt sich dabei um ein bioaktives Spezialglas, das auch für Schönheitspflegemittel und Hautpflegepräparate verwendet wird. Dieses Glas wird von der Firma ENGELHARD unter der Bezeichnung "Actysse BG" vertrieben. Das bioaktive Glas in obiger Zusammensetzung zeichnet sich besonders dadurch aus, dass es antimikrobiell gut wirksam ist. Diese Eigenschaften kommen ebenfalls der vorgeschlagenen Tätowierfarbe zugute.

Bevorzugt weist die Tätowierfarbe einen Anteil an bioaktivem Glas im Bereich von 3 Gew% bis 8 Gew% auf. Die Herstellungskosten einer Tätowierfarbe mit einem derartigen Glasanteil liegen in einem wirtschaftlich sinnvollen Bereich. Für besonders hochwertige Tätowierfarben hat sich die Verwendung von Glas im Bereich von 4,5 Gew% bis 5,5 Gew% als ideal bewährt. Insbesondere haben Optimierungsversuche bei der Herstellung der Tätowierfarbe dazu geführt, dass der Anteil von Glas an der Zusammensetzung der Farbe 5 Gew% beträgt. Bei diesem Glasanteil kommen die positiven Eigenschaften des Glases in der Tätowierfarbe am besten zur Geltung. Die übrigen Eigenschaften der Farbe, insbesondere die Farbgüte und Lichtechtheit, werden nicht beeinflusst und bleiben erhalten. Der Anteil an Povidon und Crospovidon muss bei einem fünfprozentigen Glasanteil nicht verändert werden.

Bevorzugt liegt das Glas in Form von keinen Glaspartikeln vor, die eine Größe von 0,1 µm bis 100 µm aufweisen. Aus verarbeitungstechnischer Sicht hat es sich bewährt, das bioaktive Glas in fein pulverisierter Form zuzugeben. Die Größe der Glaspartikel liegt dann deutlich unter 20 µm, vorzugsweise bei 1,5 µm. Insbesondere eine Zugabe in pudriger Form erweist sich bei der Herstellung der Tätowierfarbe als vorteilhaft. Das Glas lässt sich dann besonders einfach in die Farbe einrühren und vermischt sich sehr leicht.

Im Folgenden wird ein spezielles Ausführungsbeispiel der erfindungsgemäßen Tätowierfarbe näher beschrieben, wobei alle Bestandteile in Gewichtsprozent angegeben sind:
Die Zusammensetzung einer roten Tätowierfarbe weist die Bestandteile Wasser, Farbpigment, Povidon, Crospovidon und bioaktives Glas in Form feiner Partikel auf. Die Farbe enthält 55 Gew% demineralisiertes Wasser. Als roter Farbstoff wird das "Pigment CI 12490" verwendet. Die Tätowierfarbe hat einen Anteil an roten Farbpigmenten von 20 Gew%. Als Povidon wird "Kollidon 25" mit einem Anteil von 17,5 Gew% verwendet. Der Anteil an Crospovidon liegt bei 2,5 Gew%, um eine Verklumpung zu verhindern. Die Substanz "Kollidon CL-M" wird als Crospovidon eingesetzt. Die Glaspartikel liegen mit einem Anteil von 5 Gew% vor. Das in der Farbe enthaltene bioaktive Glas ist "Actysse BG" der Firma ENGELHARD. Diese Tätowierfarbe hat eine sehr gute Farb- und Lichtechtheit. Sie ist entzündungshemmend und fördert die Wundheilung nach der Tätowierung. Durch die antimikrobielle Wirkung des enthaltenen bioaktiven Glases wird eine Keimbildung vermieden, so dass selbst eine längere Lagerung einer bereits geöffneten Farbflasche problemlos möglich ist.

## Patentansprüche

1. Tätowierfarbe mit den folgenden Bestandteilen:
- Wasser,
- Farbpigmente,
- Bindemittel,
- bioaktives Glas.

2. Tätowierfarbe nach Anspruch 1, **dadurch gekennzeichnet, dass** ein Povidon als Bindemittel zur Verbindung der Pigmente mit dem Wasser verwendet wird, insbesondere Polyvinyl-Pyrrolidon.

3. Tätowierfarbe nach Anspruch 1 oder Anspruch 2, **dadurch gekennzeichnet, dass** die Zusammensetzung folgende Bestandteile umfasst:
35 Gew% bis 65 Gew% Wasser
1 Gew% bis 40 Gew% Farbpigment
5 Gew% bis 20 Gew% Povidon
0,1 Gew% bis 2,5 Gew% Crospovidon
0,1 Gew% bis 10 Gew% bioaktives Glas

4. Tätowierfarbe nach einem der Ansprüche 1 bis 3, **dadurch gekennzeichnet, dass** das Glas ein Gemisch folgender Zusammensetzung hat:
10 Gew% bis 90 Gew% SiO₂
0,1 Gew% bis 50 Gew% CaO
0,1 Gew% bis 60 Gew% Na₂O
0,1 Gew% bis 40 Gew% P₂O₅

5. Tätowierfarbe nach Anspruch 4, **dadurch gekennzeichnet, dass** das bioaktive Glas folgende Zusammensetzung hat:
45,0 Gew% SiO₂
24,5 Gew%CaO
24,5 Gew%Na₂O
6,0 Gew%P₂O₅

6. Tätowierfarbe nach einem der Ansprüche 1 bis 5, **dadurch gekennzeichnet, dass** der Anteil an Glas im Bereich von 3 Gew% bis 8 Gew% liegt.

7. Tätowierfarbe nach Anspruch 6, **dadurch gekennzeichnet, dass** der Anteil an Glas im Bereich von 4,5 Gew% bis 5,5 Gew% liegt.

8. Tätowierfarbe nach einem der Ansprüche 1 bis 7, **dadurch gekennzeichnet, dass** das Glas in Form von kleinen Glaspartikeln vorliegt.

9. Tätowierfarbe nach einem der Ansprüche 1 bis 8, **dadurch gekennzeichnet, dass** die Glaspartikel eine Größe von 0,1 bis 100 Mikrometern aufweisen.

10. Tätowierfarbe nach Anspruch 9, **dadurch gekennzeichnet, dass** die Glaspartikel kleiner als 20 Mikrometer sind.

## Claims

1. Tattooing ink comprising the following constituents:
- water,
- colored pigments,
- binding agents,
- bioactive glass.

2. Tattooing ink according to claim 1, **characterized in that** a binding agent for binding the pigment with the water is a povidone, in particular polyvinyl-pyrrolidone.

3. Tattooing ink according to claim 1 or 2,**characterized in that** the composition comprises the following constituents:
35 wt.% to 65 wt.% water
1 wt.% to 40 wt.% colored pigments
5 wt.% to 20 wt.% povidone
0,1 wt.% to 2,5 wt.% crospovidone
0,1 wt.% to 10 wt.% bioactive glass

4. Tattooing ink according to any one of claims 1 to 3, **characterized in that** the glass has the following composition:
10 wt.% to 90 wt.% SiO₂
0,1 wt.% to 50 wt.% CaO
0,1 wt.% to 60 wt.% Na₂O
0,1 wt.% to 40 wt.% P₂O₅

5. Tattooing ink according to claim 4, **characterized in that** the bioactive glass has the following composition:
45,0 wt.% SiO₂
24,5 wt.% CaO
24,5 wt.% Na₂O
6,0 wt.% P₂O₅

6. Tattooing ink according to any one of claims 1 to 5, **characterized in that** the content of glass is in the range of from 3 wt.% to 8 wt.%.

7. Tattooing ink according to claim 6, **characterized in that** the content of glass is in the range of from 4,5 wt.% to 5,5 wt.%.

8. Tattooing ink according to any one of claims 1 to 7, **characterized in that** the glass is present in the form of small glass particles.

9. Tattooing ink according to any one of 1 to 8, **characterized in that** the glass particles have a size of from 0,1 to 100 micrometers.

10. Tattooing ink according to claim 9, **characterized in that** the glass particles are smaller than 20 micrometers.

## Revendications

1. Couleur pour tatouage comprenant les composants suivants :
- eau,
- pigments de couleur,
- liant
- verre bio-actif.

2. Couleur pour tatouage suivant la revendication 1,
**caractérisée en ce qu'**une povidone, en particulier de la polyvinylpyrrolidone, est utilisée en tant que liant pour lier les pigments à l'eau.

3. Couleur pour tatouage suivant l'une des revendications 1 et 2, **caractérisée en ce que** la composition comprend les composants suivantes :
35% en poids à 65% en poids d'eau
1% en poids à 40% en poids de pigment de couleur
5% en poids à 20% en poids de povidone
0,1% en poids à 2,5% en poids de crospovidone
0,1% en poids à 10% en poids de verre bio-actif.

4. Couleur pour tatouage suivant l'une des revendications 1 à 3, **caractérisée en ce que** le verre comporte un mélange de composition suivants :
10% en poids à 90% en poids de SiO₂
0,1% en poids à 50% en poids de CaO
0,1% en poids à 60% en poids de Na₂O
0,1% en poids à 40% en poids de P₂O₅

5. Couleur pour tatouage suivant la revendication 4,
**caractérisée en ce que** le verre bio-actif a la composition suivante :
45,0% en poids de SiO₂
24,5% en poids de CaO
24,5% en poids de Na₂O
6,0% en poids de P₂O₅

6. Couleur pour tatouage suivant l'une des revendications 1 à 5, **caractérisée en ce que** le pourcentage de verre se situe dans la plage de 3% en poids à 8% en poids.

7. Couleur pour tatouage suivant la revendication 6,
**caractérisée en ce que** le pourcentage de verre se situe dans la plage de 4,5% en poids à 5,5% en poids.

8. Couleur pour tatouage suivant l'une des revendications 1 à 7, **caractérisée en ce que** le verre est présent en forme de petites particules de verre.

9. Couleur pour tatouage suivant l'une des revendications 1 à 8, **caractérisée en ce que** les particules de verre présentent une taille de 0,1 à 100 micromètres.

10. Couleur pour tatouage suivant la revendication 9,
**caractérisée en ce que** les particules de verre sont plus petites que 20 micromètres.
